# DEMANDE DE BREVET EUROPEEN

(11) **EP 1 118 270 A1**
(43) Date de publication de la demande: **25.07.2001**
(21) Numéro de dépôt: 01400114.3
(22) Date de dépôt: 16.01.2001
(51) Int. Cl.: A23G 3/00

(54) **Lozenges à base de dextrose et leur procédé de fabrication**

(30) Priorité: 18.01.2000 FR 0000588
(71) Demandeur: Roquette Frères, 62136 Lestrem (FR)
(72) Inventeur: Ribadeau-Dumas, Guillaume, 59237 Verlinghem (FR); Beaudier, Véronique, 59840 Lompret (FR)
(74) Mandataire: Boulinguiez, Didier

(57) **Abrégé**

L'invention a pour objet des lozenges comprenant une charge édulcorante et un liant, caractérisés par le fait que la charge édulcorante est constituée par plus de 50% de dextrose, ce pourcentage étant exprimé en poids par rapport au poids total de la charge édulcorante.

L'invention a également pour objet un procédé de préparation de tels lozenges.

## Description

L'invention a pour objet des lozenges dont la charge édulcorante est constituée majoritairement de dextrose.

Elle vise également un procédé de fabrication de ces lozenges.

Par « lozenges » on désigne des pastilles qui sont traditionnellement obtenues à partir d'une pâte à base de saccharose finement broyé. Cette pâte, qui comporte un liant assurant l'adhésion entre les cristaux de saccharose, est aromatisée, éventuellement colorée et peut également être additionnée de principes actifs pharmaceutiques, puis est amenée par laminage puis découpage à sa forme définitive de pastille que l'on sèche en vue d'éliminer l'eau qui a été nécessairement ajoutée au moment de la préparation de la pâte de départ. Les lozenges sont donc caractéristiquement obtenus sans qu'il faille avoir recours à une force de compression à la matière première de base, comme c'est le cas pour la préparation de comprimés.

Les lozenges doivent avoir une surface lisse et une texture homogène.

Le mélange intime entre le liquide, notamment l'eau de liaison et les constituants pulvérulents, à savoir le sucre et le liant, et tous autres ingrédients éventuels, peut être réalisé dans un dispositif du type pétrin ou du type extrudeur.

La pâte résultant de cette opération de mélange est amenée par laminage sous la forme d'une couche d'épaisseur choisie à l'avance et une fois que la surface de la pâte est suffisamment dure et sèche pour éviter tout collage sur les outils de découpage, on découpe les pastilles à la forme voulue avant de procéder à leur séchage. Cette étape de découpage est appelée estampage. En ce qui concerne le durcissement, il est généralement obtenu par soufflage d'air chaud et/ou froid suivi éventuellement de saupoudrage à l'aide, par exemple d'amidon, de fécule ou de mannitol.

Le séchage est généralement effectué dans des étuves à atmosphère contrôlée, afin de favoriser une texture uniforme du produit fini, les températures de séchage étant en général inférieures à 50°C et la durée du séchage inférieure à 72 heures.

En ce qui concerne la charge édulcorante, celle-ci est généralement constituée de saccharose, en mélange avec de petites quantités de dextrose ou de sirop de glucose, et parfois du sorbitol ou du glycérol à titre d'humectants retardant le séchage de façon à obtenir une surface régulière.

Il existe des lozenges « sans sucre » dans lesquels le saccharose, le dextrose, les sirops de glucose sont remplacés par des polyols tels que le xylitol, le mannitol, les hydrolysats d'amidon hydrogénés.

On peut citer notamment le brevet EP 530 995 qui décrit des lozenges dont la charge édulcorante est constituée uniquement par du maltitol ou de l'érythritol.

On a également proposé de remplacer, pour obtenir des lozenges « sans sucre » le saccharose par du sorbitol (Confectionery Production, mai 1971, pp 289-291).

La Demanderesse a également proposé dans le brevet US 4.620.982 des lozenges à base de sorbitol ou de fructose cristallisé.

Les lozenges à base de sucre sont le plus souvent formulés avec du saccharose de granulométrie très fine, comme le sucre glace. L'ajout de petites quantités de sirop de glucose améliore la finesse de la pâte, mais retarde le séchage de celle-ci. On peut également ajouter de petites quantités de dextrose, qui a l'avantage lorsqu'il est utilisé en poudre très fine de procurer au lozenge un effet rafraîchissant, ce qui est particulièrement intéressant lorsque l'on utilise des arômes menthe dans les lozenges ( LEES R., JACKSON E.B., Sugar Confectionery and Chocolate manufacture, pp 29 -295, 1973) .

Pour des raisons économiques, on a ensuite tenté de remplacer le saccharose par le dextrose dans les formules de lozenges. Mais la substitution du saccharose par le dextrose ne peut se faire qu'à hauteur d'environ 50%. En effet, lorsque la teneur en dextrose de la charge édulcorante du lozenge excède environ 50% en poids, on ne peut pas obtenir une surface aussi lisse que celle obtenue avec une charge édulcorante contenant 100% de saccharose. La pâte est très molle et collante au pétrissage, le laminage est donc plus difficile ainsi que l'estampage. Les lozenges sont de plus très friables. Une substitution satisfaisante du saccharose par le dextrose ne pouvait donc se faire qu'à hauteur d'un tiers environ.

Cherchant alors à développer des lozenges dont la charge édulcorante contient majoritairement du dextrose, la Demanderesse a eu le mérite de trouver après de nombreux essais, qu'il était possible contre toute attente de réaliser de tels lozenges, aussi lisses et de dureté comparable aux lozenges contenant majoritairement du saccharose.

L'invention a donc pour objet des lozenges comprenant une charge édulcorante et un agent liant, caractérisées par le fait que la charge édulcorante est constituée par plus de 50% de dextrose, ce pourcentage étant exprimé en poids par rapport au poids total de la charge édulcorante.

De préférence, ladite charge édulcorante est constituée par une teneur en dextrose supérieure à 60%, plus préférentiellement supérieure à 75% et plus préférentiellement encore supérieure à 90%, ces pourcentages étant exprimés en poids par rapport au poids total de charge édulcorante. Parmi les qualités de dextrose convenant bien, on peut citer notamment le dextrose monohydrate et en particulier le ROFEROSE® SF commercialisé par la Demanderesse. Le complément à 100% est avantageusement constitué par du saccharose.

En ce qui concerne la proportion de la charge édulcorante, celle-ci représente de préférence au moins 80% en poids du poids total du lozenge.

L'agent liant peut être constitué par des gommes naturelles, telles que la gomme arabique ou adragante, de la gélatine, de l'amidon, ou leurs mélanges. L'agent liant est avantageusement utilisé à raison de 0,5 à 8 % en poids, par rapport au poids total du lozenge.

Pour la préparation de lozenges selon l'invention, on préfère utiliser la gélatine, en solution aqueuse.

En ce qui concerne la teneur en eau après séchage des lozenges selon l'invention, celle-ci est avantageusement supérieure ou égale à 5% et de préférence supérieure ou égale à 7% en poids par rapport au poids total du lozenge. De telles teneurs en eau sont inattendues, les lozenges traditionnels au saccharose contenant en effet des teneurs en eau de l'ordre de 1%. Ceci est par ailleurs un avantage économique certain pour les confiseurs. Les lozenges conformes à l'invention sont de manière surprenante tout à fait satisfaisants en ce qui concerne leur dureté et leur état de surface.

Les lozenges selon l'invention sont susceptibles d'être obtenus selon un procédé comprenant les étapes de mélange de la charge édulcorante pulvérulente avec au moins un agent liant en solution, pétrissage, laminage, estampage de la pâte obtenue sous forme de lozenges et séchage des lozenges obtenus, caractérisé en ce que l'agent liant est introduit en plusieurs fois dans la charge édulcorante.

La charge édulcorante pulvérulente est introduite dans un pétrin, qui peut être chauffé ou non. Un pétrin convenant bien est un mélangeur à bras en Z. L'agent liant en solution est ensuite ajouté à la charge édulcorante. La Demanderesse a constaté que de manière surprenante et inattendue, le fait d'ajouter l'agent liant en plusieurs fois constituait un moyen propre à conférer l'excellent état de surface des lozenges conformes à l'invention. Avantageusement, l'agent liant est ajouté en deux fois. Comme pour la préparation des lozenges au saccharose, la pâte peut être additionnée d'agents aromatisants, de principes actifs pharmaceutiques, de colorants et éventuellement d'acides. L'ensemble est mélangé intimement puis la pâte est laminée, estampée et séchée dans une étuve à 30 - 50°C, pendant 8 à 24 heures. On obtient alors des lozenges d'une qualité tout à fait comparable à des lozenges au saccharose.

Les avantages de la présente invention seront mieux compris à la lecture de l'exemple suivant.

### Exemple 1 : Préparation de lozenges conformes à l'invention.

| Formule : | |
|---|---|
| Dextrose monohydraté ROFEROSE® SF | 83% en poids |
| Solution de gélatine type A 180 blooms à 10% de matière sèche | 17% en poids |

On introduit le dextrose monohydraté dans un pétrin à bras en Z non chauffé.

On ajoute la moitié de la solution de gélatine, et on pétrit pendant 6 minutes.

On ajoute l'autre moitié de la solution de gélatine et on pétrit à nouveau 6 minutes.

On lamine ensuite la pâte à 8 millimètres d'épaisseur.

On procède à l'estampage de cette pâte, qui est ensuite séchée pendant 24 heures à 20°C et 50% d'humidité relative.

On obtient des lozenges contenant 88,9% en poids de charge édulcorante (dextrose), 2% en poids de liant (gélatine) et 9,1% en poids d'eau. La charge édulcorante est constituée par 100% de dextrose.

Ces lozenges sont aussi blancs, aussi lisses et aussi durs que des lozenges contenant majoritairement du saccharose.

## Revendications

1. Lozenges comprenant une charge édulcorante et un agent liant, caractérisés par le fait que la charge édulcorante est constituée par plus de 50% de dextrose, ce pourcentage étant exprimé en poids par rapport au poids total de la charge édulcorante.

2. Lozenges selon la revendication 1, caractérisés en ce que la teneur en dextrose au sein de la charge édulcorante est supérieure à 60%, de préférence supérieure à 75% et plus préférentiellement encore supérieure à 90%, ces pourcentages étant exprimés en poids par rapport au poids total de la charge édulcorante.

3. Lozenges selon l'une ou l'autre des revendications 1 et 2, caractérisés par le fait que la charge édulcorante représente au moins 80% en poids du poids total des lozenges.

4. Lozenges selon l'une quelconque des revendications 1 à 3, caractérisés par le fait que l'agent liant est la gélatine.

5. Lozenges selon l'une quelconque des revendications 1 à 4, caractérisés par le fait que l'agent liant représente 0,5 à 8 % en poids du poids total de produit fini.

6. Lozenges selon l'une quelconque des revendications 1 à 5, caractérisés par le fait qu'ils présentent une teneur en eau supérieure ou égale à 5% et de préférence supérieure ou égale à 7% en poids par rapport au poids total de produit fini.

7. Lozenges comprenant une charge édulcorante et un liant, caractérisés en ce que la charge édulcorante est constituée par 100% de dextrose.

8. Procédé pour préparer des lozenges selon l'une quelconque des revendications 1 à 7, comprenant les étapes de mélange de la charge édulcorante avec au moins un agent liant, pétrissage, laminage, estampage sous forme de lozenges et séchage des lozenges obtenus, caractérisé en ce que l'agent liant est introduit en plusieurs fois dans la charge édulcorante.
